# EUROPEAN PATENT APPLICATION

(11) **EP 2 905 028 A1**
(43) Date of publication of application: **12.08.2015**
(21) Application number: 14154383.5
(22) Date of filing: 07.02.2014
(51) Int. Cl.: A61K 36/752, A61K 36/77, A61K 36/8962, A61K 36/185

(54) **Method of neutralizing the odor of onion**

(71) Applicant: Legacy Healthcare Holding Ltd., Valleta VLT 1165 (MT)
(72) Inventor: Harti, Saad, 1095 LUTRY (CH)
(74) Representative: Hirsch & Associés

(57) **Abstract**

The invention relates to a method of neutralizing the odor of onion of compositions comprising onion, among other components, said method comprising plunging the onion into an alcohol solution, grinding the onion plunged into the alcohol solution, filtering the grinded onion and incorporating the filtrate of onion into a composition, to obtain a composition comprising onion substantially without any olfactory note of onion.

## Description

### FIELD OF THE INVENTION

The invention relates to a method of neutralizing the odor of onion of compositions comprising onion, among other components, said method comprising plunging the onion into an alcohol solution, grinding the onion plunged into the alcohol solution, filtering the grinded onion and incorporating the filtrate of onion into a composition, to obtain a composition comprising onion substantially without any olfactory note of onion.

### BACKGROUND OF THE INVENTION

Once cut, onion is well known for freeing immediately several chemical components. Among those, propanethial S-oxide is well known for its lacrimal properties, and propanethiol is supposed to be responsible for onion characteristic odor. Indeed, when onions are grinded and the tissue is damaged, chemical reactions occur giving rise to the characteristic sulfurous note of the onion odor.

The odor of onion is very unpleasant for humans. Thus, if a composition comprises onion, the handling of said composition could be uncomfortable for the user.

For example, onion may be incorporated in cosmetic or medical compositions. Once added to a given composition, the characteristic odor of onion remains and can be, at the best, partially hidden, thanks to the addition of fragrances.

Document WO 2008/113912 describes compositions comprising an extract of *Allium species,* an extract of *Citrus species,* an extract of *Paullinia species* and an extract of *Theobroma species.* The compositions obtained according to said document show a remaining odor of onion.

Thus, there is a need to provide a method which can reduce, or even supress, the odor of onion of compositions comprising onion.

### SUMMARY OF THE INVENTION

The present invention relates to a method of neutralizing the odor of onion of a composition comprising onion, said method comprising the steps of:
a) plunging completely the onion into an alcohol solution,
b) grinding the onion plunged into the alcohol solution in order to obtain a semi-liquid homogeneous mixture,
c) filtering the semi-liquid homogeneous mixture, and
d) incorporating the filtrate obtained at the end of step c) into a composition, to obtain a composition comprising onion substantially without any olfactory note of onion.

According to an embodiment of the invention, the method further comprises a step of macerating the grinded onion into an alcohol solution before the step c).

Preferably, the onion is macerated during 24 hours to 96 hours.

According to an embodiment of the invention, *Citrus Lemon* is grinded at the same time with onion.

Preferably, the alcohol content in the alcohol solution is from 5 to 99% vol/vol, preferably from 75 to 99% vol/vol, in water.

Preferably, the alcohol solution comprises ethanol in water.

Preferably, the ethanol solution is 95% or 96% or 97% or 98% or 99% of ethanol in water.

According to an embodiment, the composition further comprises an extract of *Citrus lemon,* an extract of *Paullinia cupana,* and an extract of *Theobroma cacao.*

According to an embodiment, the method of the present invention comprises the steps of:
a) plunging completely the extract of onion and the extract of *Citrus Lemon* into an alcohol solution,
b) grinding the extract of onion and the extract of *Citrus Lemon* plunged into the alcohol solution in order to obtain a semi-liquid homogeneous mixture,
   and macerating the grinded extract of onion and the grinded extract of *Citrus Lemon* into the alcohol solution,
c) filtering the semi-liquid homogeneous mixture, and
d) introducing the extract of *Paullinia Cupana* and the extract of *Theobroma Cacao* into the filtrate obtained at the end of step c), in order to obtain the composition.

According to an embodiment, the method of the present invention further comprises, after the step of macerating, a step of a solid-liquid separation of the grinded extract of onion and the grinded extract of *Citrus Lemon* from the alcohol solution of maceration.

According to an embodiment, the composition comprises:
- from 0.1 to 10% by dry weight of an extract of *Allium Cepa,*
- from 0.1 to 10% by dry weight of an extract of *Citrus Lemon,*
- from 0.05 to 2.5% by dry weight of an extract of *Paullinia Cupana,* and
- from 0.05 to 2.5% by dry weight of an extract of *Theobroma Cacao,*
   based on the total weight of the composition.

The present invention also relates to a composition comprising onion obtained from the method according to the present invention, said composition being substantially without any olfactory note of onion.

The method according to the present invention has the advantage of neutralizing the odor of onion of compositions comprising onion.

The method according to the present invention is simple and economic.

A composition comprising onion prepared according to the process of the present invention does not show the unpleasant remaining odor of onion.

Further features and advantages of the invention will appear from the following description of embodiments of the invention, given as non-limiting examples, with reference to the accompanying drawings listed hereunder.

### DETAILED DESCRIPTION OF THE INVENTION

The present invention relates to a method of neutralizing the odor of onion from a composition comprising onion, said method comprising the steps of:
a) plunging completely the onion into an alcohol solution,
b) grinding the onion plunged into the alcohol solution in order to obtain a semi-liquid homogeneous mixture,
c) filtering the semi-liquid homogeneous mixture, and
d) incorporating the filtrate obtained at the end of step c) into a composition, to obtain a composition comprising onion substantially without any olfactory note of onion.

### Alcohol solution

According to an embodiment, the alcohol solution has an alcohol content comprised between 5 and 99% vol/vol, preferably between 15 and 99% vol/vol, preferably between 25 and 99% vol/vol, preferably between 50 and 99% vol/vol, preferably between 60 and 99% vol/vol, preferably between 75 and 99% vol/vol, in water.

According to an embodiment of the invention, the alcohol solution comprises sodium chloride.

The preferred alcohol is ethyl alcohol (ethanol) and the preferred alcohol solution is 96% vol/vol of ethanol in water.

### Composition comprising onion

The invention concerns all types of onion, also named *Allium Cepa.*

The expression "substantially without any olfactory note of onion" according to the present invention, means that the user of the composition of onion could not deduce that the composition comprises onion (intensity 0 to 2).

Odor intensity can be divided into the following categories according to the intensity:
0 - no odor
1 - very weak
2 - weak
3 - distinct
4 - strong
5 - very strong
6 - intolerable

The composition of onion obtained with the method according to the present invention has preferably no odor or very weak odor of onion: category 0 ou 1.

Another method to detect and measure odor may be a dilution-to-threshold method. According to said dilution-to-threshold technique, an odor sample is diluted at different levels of dilution with odorless ambient air and the diluted samples are presented in ascending order of odor concentration. From one level to the next, the dilution rate decreases and the amount of odorous air increases.

The first few levels include the sample diluted with a large amount of odorless ambient air, such that evaluation can begin below the threshold of detection. Preferably, repeated presentations (two odorless air samples and the diluted odor sample) are made at each level of dilution. When a forced-choice method is used, a panelist, typically persons trained to conduct these evaluations, must identify the presentation that is different from the others at each level. The threshold of detection is the dilution level at which the panelist can determine a difference between the diluted and the odorless samples. After the detection threshold is reached, the panelist continues the evaluation at the next level or two to be certain the identification was not made by chance. Examples of the dilution-to-threshold methods include use of scentometery and olfactometery.

The composition of onion substantially without any olfactory note of onion may be characterized by the amount of odorant compounds originating from onion, such as propanethiol.

In order to identify and quantify the compounds responsible of odor of onion, gas chromatography-mass spectrometry (GC/MS) may be used. According to this technique, samples are commonly trapped (adsorbed) onto some type of sorbent material which concentrates compounds of interest, those compounds may then be quantified by GC/MS. Concentrations of identified compounds responsible of odor and the interactions of the identified compounds are mathematically correlated to odor measurements made using traditional methods, for example the dilution-to-threshold methods.

### Plunging

The onion is plunged into the alcohol solution in its entire form, optionally after being peeled. According to the present invention, peeling the onion only consists in removing the external dry skin and roots.

The step of plunging the onion into the alcohol solution may be performed directly into the grinder in which the onion will be grinded.

The onion is plunged into the alcohol solution in order to be completely immersed in the alcohol solution.

### Grinding

The step of grinding the onion is performed into the alcohol solution. The grinder is filled with the alcohol solution.

During the grinding, the onion is completely immersed in the alcohol solution.

According to a particular embodiment of the invention, the onion is progressively added into the grinder during the grinding. In this case, the onion added has to be completely immersed into the alcohol solution before being grinded.

According to a further embodiment, an extract of *Citrus Lemon* is plunged into the alcohol solution and is grinded at the same time as the onion.

Preferably, the grinding is performed until obtaining a semi-liquid homogeneous mixture. The obtained mixture after grinding does not contain solid parts.

The obtained mixture is ready to be filtered.

### Macerating

Once the onion, optionally with *Citrus Lemon,* is grinded, it may further be macerated into the alcohol solution. The maceration step may last from 24 to 96 hours.

According to an embodiment of the invention, the steps of plunging, grinding, incorporating and macerating if present, are carried out simultaneously with onion and *Citrus Lemon.*

### Separation

A solid-liquid separation may further be performed after the grinding of the onion or after the maceration of the onion. The separation may be performed by one or more successive filtrations, with filters from 50 microns to 0.2 microns. If several filtrations are performed, the successive filtrations are performed on filters having smaller and smaller sizes.

According to an embodiment of the invention, the alcohol content of the aqueous-alcoholic extract of *Allium Cepa* is adjusted between 25 to 35% w/w in water.

When Citrus Lemon is also present, the alcohol content of the aqueous-alcoholic extract of *Allium Cepa* and *Citrus Lemon* is preferably adjusted between 25 to 35% w/win water.

When the composition of onion comprises *Allium Cepa, Citrus Lemon, Paullinia Cupana* and *Theobroma Cacao,* the method according to the present invention comprises the following steps:
a) plunging completely the extract of onion and the extract of *Citrus Lemon* into an alcohol solution,
b) grinding the extract of onion and the extract of *Citrus Lemon* plunged into the alcohol solution in order to obtain a semi-liquid homogeneous mixture,
   optionally macerating the grinded extract of onion and the grinded extract of *Citrus Lemon* into the alcohol solution,
c) filtering the semi-liquid homogeneous mixture obtained at the end of the grinding step or of the macerating step if present, and
d) introducing the extract of *Paullinia Cupana* and the extract of *Theobroma Cacao* into the filtrate obtained at the end of step c), in order to obtain the composition.

According to an embodiment, the composition comprising onion obtained from the method of the present invention is a cosmetic composition or a medicinal composition.

According to a particular embodiment of the invention, the composition comprising onion further comprises an extract of *Citrus Lemon,* an extract of *Paullinia Cupana* and an extract of *Theobroma Cacao.*

According to an embodiment, the composition of onion comprises:
- from 0.1 to 10% by dry weight, preferably from 0.6 to 8% by dry weight, more preferably from 0.5 to 6% by dry weight of an extract of *Allium Cepa,*
- from 0.1 to 10% by dry weight, preferably from 0.3 to 8% by dry weight, more preferably from 0.3 to 6 % by dry weight of an extract of *Citrus Lemon,*
- from 0.05 to 2.5% by dry weight, preferably from 0.07 to 1.5% by dry weight, more preferably from 0.09 to 1.2% by dry weight of an extract of *Paullinia Cupana,* and
- from 0.05 to 2.5% by dry weight, preferably from 0.07 to 2% by dry weight, more preferably from 0.09 to 1.5% by dry weight of an extract of *Theobroma Cacao,*
based on the total weight of the composition.

The composition comprising onion may further comprise excipients. For example, excipients may be chosen from talc, gum arabic, lactose, starch, magnesium stearate, cocoa butter, fatty substances of animal or vegetable origin, paraffin derivatives, glycols, various wetting agents, fragrance, dispersants or emulsifiers and preservatives.

### Application of the compositions comprising onion

Compositions comprising onion (*Allium Cepa*), *Citrus Lemon, Paullinia Cupana* and *Theobroma Cacao* described above exhibit interesting properties which make them suitable for use in cosmetics.

In cosmetic applications, these compositions may be used in particular for controlling and/or stabilising any conditions affecting the skin, the scalp or other external parts of the body, and especially for slowing down the loss of hair, stimulating its growth, increasing its density, eliminating dandruff, offsetting the signs of ageing of the scalp and treating any disorders of the nails.

In particular for topical application, the suppression of the odor of onion or at least the reduction of the odor of onion is very interesting and of particular interest, when the composition comprising onion is applied.

The invention has been described with reference to preferred embodiments. However, many variations are possible within the scope of the invention.

### EXAMPLES

### Comparative example

A composition has been prepared according to Example 1 of WO 2008/113912. According to the Example 1 of WO 2008/113912, the composition is prepared according to the following process:
- preparation of the aqueous-alcoholic extract of *Allium Cepa* according to the following successive steps:
   o picking, cleaning, and grinding the *Allium Cepa* in the open air,
   o macerating the grinded *Allium Cepa* in 96% vol/vol of alcohol in water for 48 hours in the cold,
   o filtering through a 0.22-micron filter,
   o collecting the alcoholic filtrate,
   o adjusting the alcoholic filtrate to 30% w/w of alcohol in water;
- preparation of the aqueous-alcoholic extract of *Citrus Lemon* according to the same successive steps as for *Allium Cepa;*
- mixing the aqueous-alcoholic extracts of *Allium Cepa* and *Citrus Lemon;*
- addition of atomized aqueous-alcoholic extracts of *Paullinia cupana* and *Theobroma cacao.*

There is a remaining odor of onion for the composition obtained according to the method described in WO 2008/113912. For example, the user of the composition will detect that the composition contains onion. The composition containing onion obtained according to the process described in document WO 2008/113912 is characterized by the intensity 3 according to the categories from 0-6 given above.

### Example I According to the present invention

A composition has been prepared according to the following process:
- preparation of the aqueous-alcoholic extract of *Allium Cepa* according to the following successive steps:
   o peeling, cleaning the Allium Cepa in the open air,
   o plunging the *Allium Cepa* in a grinder comprising an ethyl alcohol solution 96%, such that the alcohol solution completely covers the *Allium Cepa,*
   o grinding the *Allium Cepa,*
   o macerating the grinded *Allium Cepa* in 96% vol/vol of alcohol in water for 48 hours in the cold,
   o filtering through a 0.22 microns filter,
   o collecting the alcoholic filtrate,
   o adjusting the alcoholic filtrate to 30% w/w of alcohol in water;
- preparation of the aqueous-alcoholic extract of *Citrus Lemon* according to the same successive steps as for *Allium Cepa;*
- mixing the aqueous-alcoholic extracts *of Allium Cepa* and *Citrus Lemon;*
- addition of atomized aqueous-alcoholic extracts of *Paullinia cupana* and *Theobroma cacao.*

There is no remaining odor of onion for the composition obtained with the method according to the present invention. For example, the user of the composition will not be able to detect that the composition contains onion. The composition containing onion obtained according to the process of example 1 is characterized by the intensity 0, 1 or 2 according to the categories from 0-6 given above.

## Claims

1. Method of neutralizing the odor of onion of a composition comprising onion, said method comprising the steps of:
a) plunging completely the onion into an alcohol solution,
b) grinding the onion plunged into the alcohol solution in order to obtain a semi-liquid homogeneous mixture,
c) filtering the semi-liquid homogeneous mixture, and
d) incorporating the filtrate obtained at the end of step c) into a composition, to obtain a composition comprising onion substantially without any olfactory note of onion.

2. Method according to claim 1, further comprising a step of macerating the grinded onion into an alcohol solution before the step c).

3. Method according to claim 2, wherein the onion is macerated during 24 hours to 96 hours.

4. Method according to any one of claims 1 to 3, wherein *Citrus Lemon* is grinded at the same time with onion.

5. Method according to any one of claims 1 to 4, wherein the alcohol content in the alcohol solution is from 5 to 99% vol/vol, preferably from 75 to 99% vol/vol, in water.

6. Method according to any one of claims 1 to 5, wherein the alcohol solution comprises ethanol in water.

7. Method according to claim 6, wherein the ethanol solution is 95% or 96% or 97% or 98% or 99% of ethanol in water.

8. Method according to any one of claims 1 to 7, wherein the composition further comprises an extract of *Citrus lemon,* an extract of *Paullinia cupana,* and an extract of *Theobroma cacao.*

9. Method according to claim 8, comprising the steps of:
a) plunging completely the extract of onion and the extract of *Citrus Lemon* into an alcohol solution,
b) grinding the extract of onion and the extract of *Citrus Lemon* plunged into the alcohol solution in order to obtain a semi-liquid homogeneous mixture,
and macerating the grinded extract of onion and the grinded extract of *Citrus Lemon* into the alcohol solution,
c) filtering the semi-liquid homogeneous mixture, and
d) introducing the extract of *Paullinia Cupana* and the extract of *Theobroma Cacao* into the filtrate obtained at the end of step c), in order to obtain the composition.

10. Method according to claim 9, further comprising, after the step of macerating, a step of a solid-liquid separation of the grinded extract of onion and the grinded extract of *Citrus Lemon* from the alcohol solution of maceration.

11. Method according to any one of claims 8 to 10, wherein the composition comprises:
- from 0.1 to 10% by dry weight of an extract of *Allium Cepa,*
- from 0.1 to 10% by dry weight of an extract of *Citrus Lemon,*
- from 0.05 to 2.5% by dry weight of an extract of *Paullinia Cupana,* and
- from 0.05 to 2.5% by dry weight of an extract of *Theobroma Cacao,*
based on the total weight of the composition.

12. Composition comprising onion obtained from the method according to any one of claims 1 to 11, said composition being substantially without any olfactory note of onion.
